**Europäisches Patentamt**

**European Patent Office**

**Office européen des brevets**

(19)

(11) Veröffentlichungsnummer: **0 026 494**
**A1**

(12) **EUROPÄISCHE PATENTANMELDUNG**

(21) Anmeldenummer: **80105925.4**

(22) Anmeldetag: **30.09.80**

(51) Int. Cl.³: **H 05 G 1/44**
**A 61 B 6/00**

(30) Priorität: **02.10.79 DE 2939975**

(43) Veröffentlichungstag der Anmeldung:
**08.04.81 Patentblatt 81/14**

(84) Benannte Vertragsstaaten:
**DE FR GB IT SE**

(71) Anmelder: **SIEMENS AKTIENGESELLSCHAFT Berlin und München**
**Postfach 22 02 61**
**D-8000 München 22(DE)**

(72) Erfinder: **Pfeiler, Manfred, Dr., Dipl.-Ing.**
**Ludwig-Thoma-Strasse 25**
**D-8520 Erlangen(DE)**

(54) **Röntgenschichtgerät zur Herstellung von Transversalschichtbildern.**

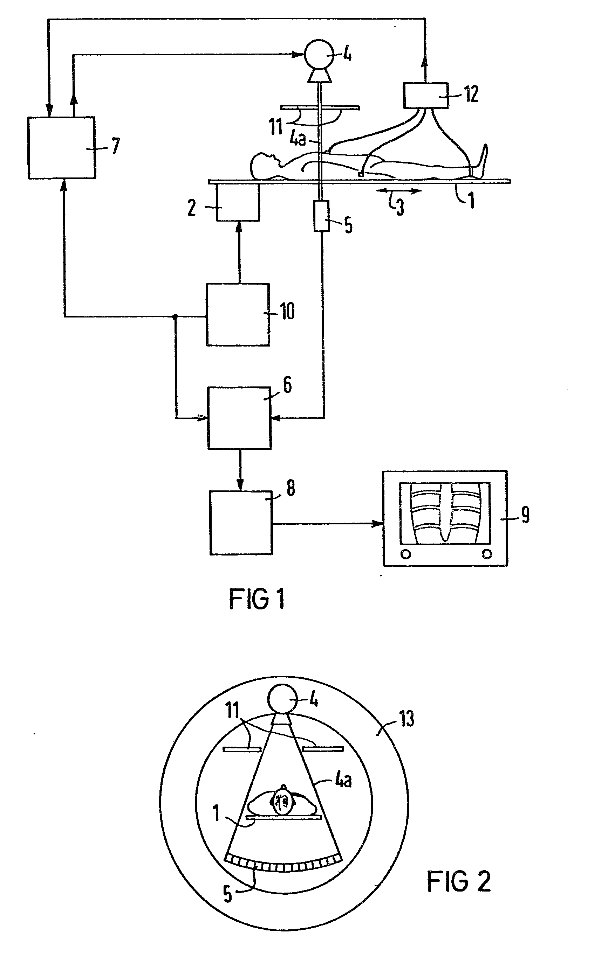

(57) Die Erfindung bezieht sich auf einen Computertomographen mit einer Röntgenstrahlen-Meßanordnung (4, 5) und einer längsverschiebbaren Patientenliege (1). Das Transversalschichtbild wird durch Drehung der Meßanordnung (4, 5) in einer senkrecht zur Patientenliege (1) liegenden Ebene erzeugt. Zur Anfertigung einer Übersichtsaufnahme wird der Patient bei arretierter Meßanordnung (4, 5) in Längsrichtung verschoben. Dabei wird bei der Verwendung eines Detektorarrays als Strahlenempfänger (5) die Röntgenröhre gepulst und der Rechner (8) speichert bei jedem Röntgenstrahlenpuls die einer Bildzeile entsprechenden Ausgangssignale des Strahlenempfängers (5). Die Einschaltung der Röntgenröhre (4) wird vom EKG synchronisiert (Fig. 1).

FIG 1

EP 0 026 494 A1

SIEMENS AKTIENGESELLSCHAFT     Unser Zeichen
Berlin und München             VPA 79 P 5100 EUR

Röntgenschichtgerät zur Herstellung von Transversalschichtbildern

Die Erfindung bezieht sich auf ein Röntgenschichtgerät zur Herstellung von Transversalschichtbildern eines Aufnahmeobjektes mit einer Patientenliege, mit einer Röntgenstrahlen-Meßanordnung mit einer von einem Röntgengenerator gespeisten, gepulsten Röntgenstrahlenquelle, die ein das Aufnahmeobjekt durchdringendes Röntgenstrahlenbündel erzeugt, dessen Querschnittsausdehnung senkrecht zur Schichtebene gleich der Schichtstärke ist und mit einem Strahlenempfänger, der die Strahlenintensität hinter dem Objekt ermittelt, mit einer Antriebsvorrichtung für die Meßanordnung mit einem Drehrahmen zur Erzeugung von Drehbewegungen der Röntgenstrahlen-Meßanordnung und mit einem Meßwertumformer für die Transformation der vom Strahlenempfänger gelieferten Signale in ein Schichtbild, bei dem Mittel zur Erzeugung einer Relativbewegung zwischen dem Röntgenstrahlenbündel und der Patientenliege in Liegenlängsrichtung während der Verarbeitung der vom

Tp 5 Ler / 19.9.1979

Strahlenempfänger gelieferten Signale bei gegen Drehung verriegelter Meßanordnung vorhanden sind, und bei dem am Meßwertumformer eine Einrichtung zum Aufbau und zur Wiedergabe des vom Meßwertumformer aus den Signalen des Strahlenempfängers bestimmten, dem Verschiebungsbereich entsprechenden Röntgenschattenbildes des Patienten angeschlossen ist.

Zur Erfassung des Schichtbildes können dabei die Drehbewegungen um kleine äquidistante Winkelbeträge in wechselnder Folge mit je einer Verschiebung der Meßanordnung längs einer zum Zentralstrahl des Röntgenstrahlenbündels senkrechten Geraden erfolgen. Es ist aber auch möglich, auf die Verschiebungen zu verzichten, wenn der Strahlenempfänger aus einer Vielzahl von Strahlendetektoren aufgebaut wird, und wenn das Röntgenstrahlenbündel fächerförmig ist und alle Detektoren gleichzeitig trifft.

In der DE-OS 26 13 809 ist ein Röntgenschichtgerät der eingangs genannten Art beschrieben, bei dem zusätzlich zu der Wiedergabe von Transversalschichtbildern auch die Wiedergabe von Röntgenschattenbildern möglich ist, die dadurch erzeugt werden, daß bei der Relativbewegung zwischen der Meßanordnung und der Patientenliege in Liegenlängsrichtung die Meßwerte des Strahlenempfängers im Meßwertumformer gespeichert und daraus das Röntgenschattenbild erzeugt wird. Wegen der erforderlichen Relativbewegung ist die Zeit für die Anfertigung eines Röntgenschattenbildes so lange, daß die Grenzen bewegter Organe, insbesondere des Herzens, in undefinierter Weise im Röntgenschattenbild dargestellt werden.

35

- 3 -        VPA 79 P 5100  EUR

Der Erfindung liegt die Aufgabe zugrunde, ein Röntgenschichtgerät der eingangs genannten Art so auszubilden, daß eine eindeutige, scharfe Abbildung bewegter Organgrenzen, insbesondere der Herzränder, erfolgt.

Diese Aufgabe ist erfindungsgemäß dadurch gelöst, daß der Röntgengenerator an einer EKG-Abnahmeeinheit angeschlossen ist, die die Einschaltung der Röntgenröhre an vorbestimmten Stellen des Herzzyklus bewirkt. Bei dem erfindungsgemäßen Röntgenschichtgerät werden alle Meßwertsätze bei bestimmten Herzphasen erzeugt, so daß die Herzränder und auch die Grenzen anderer, infolge der Herzkontraktionen bewegter Organe eindeutig und scharf im Röntgenschattenbild erscheinen.

Die Erfindung ist nachfolgend anhand eines in der Zeichnung dargestellten Ausführungsbeispiel näher erläutert. Es zeigen:

Fig. 1 die für die Erfindung wesentlichen Teile eines Röntgenschichtgerätes nach der Erfindung, und

Fig. 2 eine Einzelheit des Gerätes gemäß Figur 1.

In der Figur 1 ist eine Patientenliege 1 dargestellt, die durch einen Motor 2 in Richtung des Doppelpfeils 3 hin und her bewegbar ist. Zur Erzeugung von Röntgenbildern ist eine Meßanordnung vorhanden, die aus einer Röntgenröhre 4 und einem Strahlenempfänger 5 besteht. Der Ausgang des Strahlenempfängers 5 ist an einer Meßwertabfrageeinheit 6 angeschlossen, die einen Röntgengenerator 7 und einen Rechner 8 steuert. Der Ausgang des Rechners 8 ist an einem Fernsehsichtgerät 9 angeschlossen. Ferner ist eine Steuervorrichtung 10 für

den Liegemotor 2 vorhanden, die auch am Röntgengenerator 7 und an der Meßwertabfrageeinheit 6 angeschlossen ist.

Die Meßanordnung 4, 5 ist gemäß Figur 2 auf einem Drehrahmen 13 angeordnet und weist eine Röntgenröhre 4 auf, welche ein durch eine Blende 11 eingeblendetes fächerförmiges Röntgenstrahlenbündel 4a erzeugt, dessen Stärke senkrecht zur durchstrahlten Schicht gleich der Schichtstärke ist und das auf dem um den Fokus der Röntgenröhre 4 gekrümmten Strahlenempfänger 5 auftrifft. Der Strahlenempfänger 5 besteht aus einer Detektorreihe mit einer Vielzahl von Detektoren, beispielsweise 256 Detektoren, so daß bei der Durchstrahlung des Patienten bei einer bestimmten Projektion 256 einzelne Meßwerte gewonnen werden. Zur Erzeugung eines Transversalschichtbildes wird die Einheit 4, 5 mittels des Drehrahmens 13 um den Patienten in einer senkrecht zur Patientenliege 1 liegenden Ebene, und zwar in der Ebene des Röntgenstrahlenbündels 4a, gedreht. Die in vorbestimmten Stellungen der Meßanordnung 4, 5 gelieferten Ausgangssignale des Strahlenempfängers 5 werden von der Meßwertabfrageeinheit 6 dem Rechner 8 zugeführt, der daraus in bekannter Weise ein Transversalschichtbild in Form einer Matrix von Schwächungskoeffizienten berechnet.

Zur Erzeugung eines Röntgenschattenbildes wird die Einheit 4, 5 arretiert und die Liege 1 mit dem Patienten um ein vorbestimmtes Maß in ihrer Längsrichtung verschoben. Während der Verschiebung wird die Röntgenröhre 4 gepulst und für jeden Röntgenstrahlenpuls der Strahlenempfänger 5 abgefragt. Man erhält daher für vorbestimmte Stellungen der Liege 1 mit dem Patienten Meßwerte, die die Schwächung der Röntgenstrahlung beim Durchtritt durch den Patienten charakterisieren. Der Rechner 8 oder ein anderes geeignetes Mittel zur Bestimmung des Röntgenschattenbildes bestimmt daraus das Röntgenschattenbild und bewirkt seine Wiedergabe auf dem Sichtgerät 9. Jeder Bildzeile des auf dem Sichtgerät 9 wiedergegebenen Bildes entspricht dabei eine Abfrage des Strahlenempfängers 5 und die Anzahl der effektiven Bildpunkte pro Bildzeile ist gleich der Anzahl der Detektoren im Strahlenempfänger 5. Die Pulsfolge und die Geschwindigkeit, mit der die Liege 1 mit dem Patienten bewegt wird, sind möglichst so aufeinander abgestimmt, daß die örtliche Auflösung in Längsrichtung des Patienten der entspricht, die durch die Zahl der Detektoren im Strahlenempfänger 5 in Querrichtung erreicht werden kann.

Im Rahmen der Erfindung ist es auch bedingt möglich, anstelle einer Detektorreihe mit vielen Detektoren einen einzigen Detektor oder eine geringe Anzahl von Detektoren als Strahlenempfänger zu benutzen, wenn die Einheit 4, 5 quer zum Patienten und zur Liege 1 verschiebbar angeordnet wird.

Das Pulsen der Röntgenröhre 4 erfolgt bei dem beschriebenen Ausführungsbeispiel in vorbestimmten Stellungen der Patientenliege 1, d.h. in vorbestimmten Stellungen der Liege 1 wird der Röntgengenerator 7 an die Röntgenröhre 4 angeschaltet. Die periodische Einschaltung der Röntgenröhre 4 ist mit dem Elektrokardiogramm synchronisiert. Hierzu ist eine EKG-Abnahmeeinheit 12 mittels Elektroden am Patienten angeschlossen. Die EKG-Abnahmeeinheit 12 steuert den Röntgengenerator 7 so, daß die Einschaltung der Röntgenröhre an einer oder mehreren vorbestimmten Stellen des Herzzyklus erfolgt. Auf diese Weise erhält man ein oder mehrere Röntgenschattenbild(er), in dem (denen) die Herzränder und die Grenzen anderer, aufgrund der Herzkontraktionen bewegter Organe scharf abgebildet sind, denn sie werden immer dann durchstrahlt, wenn sie eine für alle Durchstrahlungen gleiche Lage einnehmen.

Im Rahmen der Erfindung ist es auch denkbar, die Liege 1 ortsfest zu machen und die Meßanordnung 4, 5 sowohl zur Herstellung einer Übersichtsaufnahme als auch zur Schichtwahl für ein Transversalschichtbild in Längsrichtung der Liege 1 verschiebbar vorzusehen.

Der Rechner 8 enthält einen Speicher, der die einer Bildzeile entsprechenden, vom Strahlenempfänger 5 gelieferten Signale speichert, bis der gewünschte Bereich des Patienten durch Längsverschiebung der Liege 1 abgetastet und damit das gewünschte Übersichtsbild im Rechner 9 gespeichert ist und auf dem Fernsehsichtgerät 9 wiedergegeben werden kann.

Patentanspruch

Röntgenschichtgerät zur Herstellung von Transversalschichtbildern eines Aufnahmeobjektes mit einer Patientenliege, mit einer Röntgenstrahlen-Meßanordnung mit
einer von einem Röntgengenerator gespeisten, gepulsten
Röntgenstrahlenquelle, die ein das Aufnahmeobjekt
durchdringendes Röntgenstrahlenbündel erzeugt, dessen
Querschnittsausdehnung senkrecht zur Schichtebene
gleich der Schichtstärke ist und mit einem Strahlenempfänger, der die Strahlenintensität hinter dem Objekt
ermittelt, mit einer Antriebsvorrichtung für die Meßanordnung mit einem Drehrahmen zur Erzeugung von Drehbewegungen der Röntgenstrahlen-Meßanordnung und mit einem
Meßwertumformer für die Transformation der vom Strahlenempfänger gelieferten Signale in ein Schichtbild,
bei dem Mittel zur Erzeugung einer Relativbewegung zwischen dem Röntgenstrahlenbündel und der Patientenliege
in Liegenlängsrichtung während der Verarbeitung der
vom Strahlenempfänger gelieferten Signale bei gegen
Drehung verriegelter Meßanordnung vorhanden sind, und
bei dem am Meßwertumformer eine Einrichtung zum Aufbau
und zur Wiedergabe des vom Meßwertumformer aus den Signalen des Strahlenempfängers bestimmten, dem Verschiebungsbereich entsprechenden Röntgenschattenbildes des
Patienten angeschlossen ist, d a d u r c h  g e -
k e n n z e i c h n e t ,  daß der Röntgengenerator
(7) an einer EKG-Abnahmeeinheit (12) angeschlossen
ist, die die Einschaltung der Röntgenröhre (4) an vorbestimmten Stellen des Herzzyklus bewirkt.

0026494

1/1

FIG 1

FIG 2

Europäisches
Patentamt

**EUROPÄISCHER RECHERCHENBERICHT**

Nummer der Anmeldung

EP 80105925.4

| | EINSCHLÄGIGE DOKUMENTE | | | KLASSIFIKATION DER ANMELDUNG (Int Cl ) |
|---|---|---|---|---|
| Kategorie | Kennzeichnung des Dokuments mit Angabe, soweit erforderlich, der maßgeblichen Teile | | betrifft Anspruch | |
| | DE - B - 1 015 155 (SIEMENS) + Anspruch 1; Spalte 3, Zeilen 4-17; Fig. 4 + | | 1 | H 05 G 1/44 A 61 B 6/00 |
| | -- | | | |
| | DE - C - 939 521 (ATLAS) + Seite 2, Zeilen 1-15 + | | 1 | |
| | -- | | | |
| | DE - C - 935 263 (ATLAS) + Seite 1, Zeilen 1-17; Anspruch 1 + | | 1 | |
| | -- | | | RECHERCHIERTE SACHGEBIETE (Int Cl ) |
| | DE - C - 305 289 (SIEMENS) + Seite 1, Zeilen 1-64; Seite 2, Zeilen 1-31; Anspruch 1 + | | 1 | H 05 G 1/00 A 61 B 6/00 G 03 B 41/00 |
| | -- | | | |
| | US - A - 3 626 932 (BECKER) + Seiten 1,2; Anspruch 1 + | | 1 | |
| | -- | | | |
| A | DE - B2 - 2 462 239 (SIEMENS) + Gesamt + | | 1 | |
| | -- | | | |
| A | DE - A1 - 2 436 696 (BRATTLE) + Gesamt + | | 1 | KATEGORIE DER GENANNTEN DOKUMENTE |
| | -- | | | X: von besonderer Bedeutung |
| A | DE - A1 - 2 813 830 (OHIO NUCLEAR) + Gesamt + | | 1 | A: technologischer Hintergrund O: nichtschriftliche Offenbarung P: Zwischenliteratur T: der Erfindung zugrunde |
| | -- | | | liegende Theorien oder |
| D,A | DE - B2 - 2 613 809 (SIEMENS) + Gesamt + | | 1 | Grundsätze E: kollidierende Anmeldung D: in der Anmeldung angeführtes Dokument L: aus andern Gründen angeführtes Dokument |
| | ---- | | | |
| | | | | &: Mitglied der gleichen Patent- |
| X | Der vorliegende Recherchenbericht wurde für alle Patentansprüche erstellt. | | | familie, übereinstimmendes Dokument |

| Recherchenort | Abschlußdatum der Recherche | Prüfer |
|---|---|---|
| WIEN | 22-12-1980 | VAKIL |